# EUROPEAN PATENT SPECIFICATION

(11) **EP 4 473 992 B1**
(45) Date of publication and mention of the grant of the patent: **12.08.2026**
(21) Application number: 24180101.8
(22) Date of filing: 05.06.2024
(51) Int. Cl.: A61M 25/00, A61N 1/362, A61N 1/372, A61N 1/39

(54) **WIRELESS CARDIAC STIMULATION**
DRAHTLOSE HERZSTIMULATION
STIMULATION CARDIAQUE SANS FIL

(30) Priority: 06.06.2023 US 202318206212
(43) Date of publication of application: 11.12.2024
(73) Proprietor: Biosense Webster (Israel) Ltd., 2066717 Yokneam (IL)
(72) Inventor: GOVARI, Assaf, 2066717 Yokneam (IL); GLINER, Vadim, 2066717 Yokneam (IL)
(74) Representative: Carpmaels & Ransford LLP

(56) References cited:
- US-A1- 2012 172 891
- US-A1- 2013 023 746
- US-A1- 2016 114 169
- US-A1- 2016 183 793

## Description

### FIELD OF THE DISCLOSURE

The present disclosure relates to medical devices, and in particular, but not exclusively, to cardiac stimulation.

### BACKGROUND

A wide range of medical procedures involve placing probes, such as catheters, within a patient's body. One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Catheters are inserted into the heart chamber and optionally around the heart chamber during such procedures. In most procedures, multiple catheters are inserted into the patient. Catheters may include mapping, ablation, temperature sensing and image sensing catheters. Some catheters are dedicated for placement in specific parts of the anatomy, e.g., coronary sinus, esophagus, atrium, ventricle. The catheters have multiple electrical channels, some more than others depending on the number of sensors and electrodes included in each catheter. The number and type of catheters depends on the procedure and on the physician preferred workflow.

US2016183793A1 describes a Wireless Catheter Module (WCM) adapted for attachment to a diagnostic catheter to enable the bio-electrical signals, analog signals picked up by the probing electrodes imbedded in the attached catheter, to be captured and digitized by A-to-D converter electronics in the WCM whereupon packets of the digitized signals can then be transmitted wirelessly from the WCM to a remote Wireless Base Station (WBS) forming a receiver component of the present system. The WBS is adapted for operative connected to an EP recording and cardiac stimulation system. In cases where multiple catheters are to be used in a study, each catheter may be fitted with an appropriately configured WCM and the catheter modules can communicate simultaneously with the WBS.

### BRIEF DESCRIPTION OF THE DRAWINGS

The present disclosure will be understood from the following detailed description, taken in conjunction with the drawings in which:
Fig. 1 is a pictorial view of a catheter-based electrophysiology mapping and ablation system constructed and operative in accordance with an exemplary mode of the present disclosure;
Figs. 2-5 are block diagram views of alternative pacing subsystems in the system of Fig. 1; and
Fig. 6 is a block diagram view of a defibrillation subsystem in the system of Fig. 1.

### DESCRIPTION OF EXAMPLES

The present invention is defined by the appended claims, and examples described hereinafter are presented for better understanding the present invention. One or more catheters are typically connected via one or more cables to a patient interface unit (PIU) which may perform some signal processing tasks and compute the position of the catheter or send data to another processing device for further processing. One drawback of the above system is that the cable(s) connecting the PIU with the catheter(s) are often an obstacle in the electrophysiological (EP) procedure room. The cable(s) may be a tripping hazard leading to accidents. In some cases, the cable(s) may become disconnected from the PIU leading to malfunctioning of the system.

One solution is to provide a wireless link between the PIU and the catheter(s). Providing a wireless link removes the cable and solves the abovementioned drawbacks, but leads to further challenges regarding how to perform pacing and/or defibrillation with the catheter(s) when these functions are normally initiated and driven by the PIU or other devices such as a pacing device or recording system.

Exemplary modes of the present disclosure solve at least some of the above drawbacks by providing a wireless catheter device including a signal generator which is driven either by a digitized pacing signal or pacing parameters wirelessly received from a PIU, or recording system, or any suitable device(s).

In some exemplary modes, a device such as the PIU, or a personal computer (e.g., running EP software such as CARTO^{™} 3), or a recording system, receives one or more pacing parameters from a user, e.g., physician. The pacing parameter(s) is saved by the device until the user initiates pacing. The pacing parameters may include any one or more of the following: a pacing signal frequency; a pacing signal duty cycle; a pacing signal amplitude; a pacing signal pulse shape; a pacing signal pulse length; and/or a given electrode of a catheter to which to apply pacing. When the user decides to initiate pacing, the user makes an appropriate selection via a user interface of the device and the device wirelessly sends the pacing parameter(s) and optionally a command to initiate pacing to a catheter device. The signal generator of the catheter device generates a pacing signal according to the pacing parameter(s) and applies the pacing signal to the selected catheter electrode. The timing of the pacing signal pulses may be synchronized with the heart rhythm of the patient. For example, the signal generator may synchronize the pulses of the pacing signal based on intracardiac electrograms (IEGMs) received from electrodes of a catheter in the cardiac chamber of the patient. For example, the pacing system may look for the diastolic phase in the IEGMs in which to time the pacing pulses. Similarly, in the other exemplary modes described below, the timing of the pacing pulses may be synchronized with the heart rhythm of the patient.

In some exemplary modes, a device such as a recording system or pacing device generates a first pacing signal based on user input for outputting to a wired port of the device or to the wired port of another device (e.g., the PIU), for example, using a legacy pacing subsystem in the device. The recording system or another device (e.g., PIU or PC) detects the generated first pacing signal and in response wirelessly sends a command to the catheter device to generate a second pacing signal according to the same parameters used to generate the first pacing signal.

In some exemplary modes, a device such as the PIU, or a personal computer (e.g., running EP software such as CARTO^{™} 3), or a recording system, receives one or more pacing parameters from a user, e.g., physician. The pacing parameter(s) may be saved by the device until the user initiates pacing. When the user decides to initiate pacing, the user makes an appropriate selection via a user interface of the device and the device generates a pacing signal according to the pacing parameter(s). The device wirelessly sends a digitized version of the pacing signal to the catheter device. The signal generator of the catheter device mimics the received digitized pacing signal (by performing a digital to analog conversion and optionally amplification) and applies the mimicked pacing signal to the selected catheter electrode.

In some exemplary modes, a device such as a recording system or pacing device generates a pacing signal based on user input for outputting to a wired port of the device or to the wired port of another device (e.g., the PIU), for example, using a legacy pacing subsystem in the device. The recording system or another device (e.g., PIU or PC) detects the generated pacing signal and in response wirelessly sends a digitized version of the pacing signal to the catheter device. The signal generator of the catheter device mimics the received digitized pacing signal (by performing a digital to analog conversion and optionally amplification) and applies the mimicked pacing signal to the selected catheter electrode.

Typically, in EP procedures defibrillation is delivered through patches on the skin. In such a case, a strong current is used to affect the depolarization needed. Therefore, in some exemplary modes, a wireless catheter performs intracardiac defibrillation. Since the electrical current is delivered locally by the catheter electrodes, there is no need for such a strong signal. Instead, the catheter electrodes can affect the depolarization needed with a relatively low gain signal that can be generated for example by a signal generator included in an ASIC attached to the catheter. The catheter includes a signal generator, which is triggered by a signal processing unit to generate a defibrillation signal (e.g., a pulse) in response to the signal processing unit identifying a cardiac event that indicates that defibrillation should be performed. The signal processing unit may identify the cardiac event based on intracardiac electrograms (IEGMs) captured by the wireless catheter or by another catheter (wireless or wired) and received by the signal processing unit. In some exemplary modes, the signal processing unit is installed in the wireless catheter. In other exemplary modes, the signal processing unit is installed in a device remote to the wireless catheter such as the PIU. When the signal processing unit is installed in a device remote to the wireless catheter, the IEGMs are typically digitized by the wireless catheter and sent to the signal processing unit for analysis.

### SYSTEM DESCRIPTION

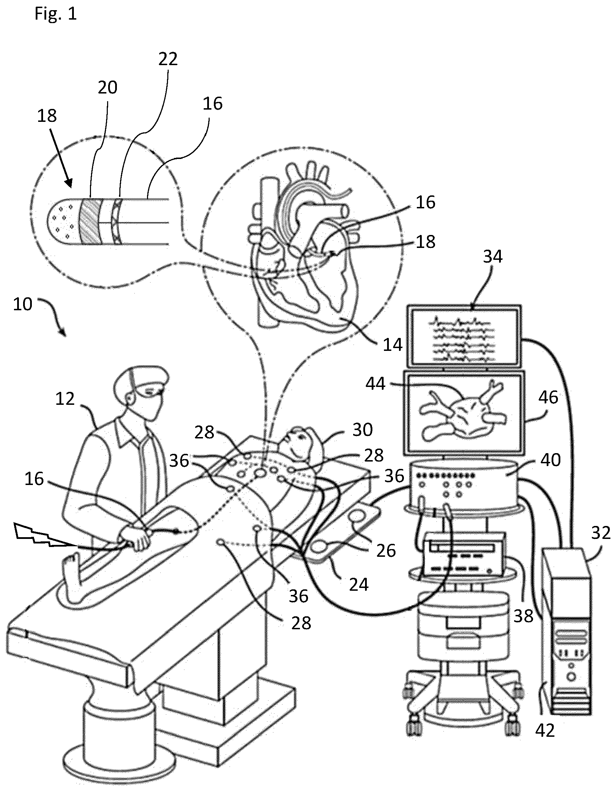

Reference is made to Fig. 1, which is a pictorial view of a catheter-based electrophysiology mapping and ablation system 10 constructed and operative in accordance with an exemplary mode of the present disclosure. System 10 includes multiple catheters, which are percutaneously inserted by a physician 12 through the patient's vascular system into a chamber or vascular structure of a heart 14. Typically, a delivery sheath catheter is inserted into the left or right atrium near a desired location in heart 14. Thereafter, one or more catheters may be inserted into the delivery sheath catheter so as to arrive at the desired location in heart 14. The plurality of catheters may include catheters dedicated for sensing Intracardiac Electrogram (IEGM) signals, catheters dedicated for ablating and/or catheters dedicated for both sensing and ablating. An example catheter 16 that is configured for sensing IEGMs and performing ablation is illustrated herein. Physician 12 may place a distal tip 18 of catheter 16 in contact with the heart wall for sensing a target site in heart 14. For ablation, physician 12 may similarly place the distal tip 18 of the catheter 16 in contact with a target site for ablating tissue.

Catheter 16 is an exemplary catheter that includes one or more electrodes 20 optionally distributed over the distal tip 18 and configured to sense the IEGM signals. Catheter 16 may additionally include a position sensor 22 embedded in or near distal tip 18 for tracking position and orientation of distal tip 18. Position sensor 22 may be a magnetic based position sensor including three magnetic coils for sensing three-dimensional (3D) position and orientation (including roll).

Magnetic based position sensor 22 may be operated together with a location pad 24 including a plurality of magnetic coils 26 configured to generate magnetic fields in a predefined working volume. Real time position of distal tip 18 of catheter 16 may be tracked based on magnetic fields generated with location pad 24 and sensed by magnetic based position sensor 22. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode (body surface) patches 28 positioned for skin contact on patient 30 to establish location reference for location pad 24 as well as impedance-based tracking of electrode(s) 20. For impedance-based tracking, electrical current is directed to electrode(s) 20 and sensed at electrode body surface patches 28 so that the location of each electrode can be triangulated (or otherwise computed) via the electrode patches 28. Details of the impedance-based location tracking technology are described in US Patent Nos. 7,536,218; 7,756,576; 7,848,787; 7,869,865; and 8,456,182.

A recorder 32 (e.g., recording subsystem) records and displays electrograms 34 captured with body surface ECG electrodes 36 and intracardiac electrograms (IEGM) captured with electrode(s) 20 of catheter 14. Recorder 32 may include pacing capability for pacing the heart rhythm and/or may be electrically connected to a standalone pacer.

System 10 may include an ablation energy generator 38 that is adapted to conduct ablative energy to the electrode(s) 20 at the distal tip 18 of the catheter 16 configured for ablating. Energy produced by ablation energy generator 38 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 40 is an interface configured to establish electrical communication between catheters, other electrophysiological equipment, power supply and a workstation 42 (e.g., personal computer) for controlling operation of system 10. Electrophysiological equipment of system 10 may include for example, multiple catheters, location pad 24, body surface ECG electrodes 36, electrode patches 28, ablation energy generator 38, and recorder 32. Optionally and preferably, PIU 40 additionally includes processing capability for implementing real-time computations of location of the catheters and for performing ECG calculations.

Workstation 42 includes a memory, a processor unit with a memory or storage with appropriate operating software stored therein, and user interface capability. Workstation 42 may provide multiple functions, optionally including (1) modeling the endocardial anatomy in three-dimensions (3D) and rendering a model or anatomical map 44 for display on a display device 46, (2) displaying on display device 46 activation sequences (or other data) compiled from recorded electrograms 34 in representative visual indicia or imagery superimposed on the rendered anatomical map 44, (3) displaying real-time location and orientation of multiple catheters within the heart chamber, and (4) displaying on display device 46 sites of interest such as places where ablation energy has been applied. One commercial product embodying elements of the system 10 is available as the CARTO^{™} 3 System, available from Biosense Webster, Inc., 31A Technology Drive, Irvine, CA 92618.

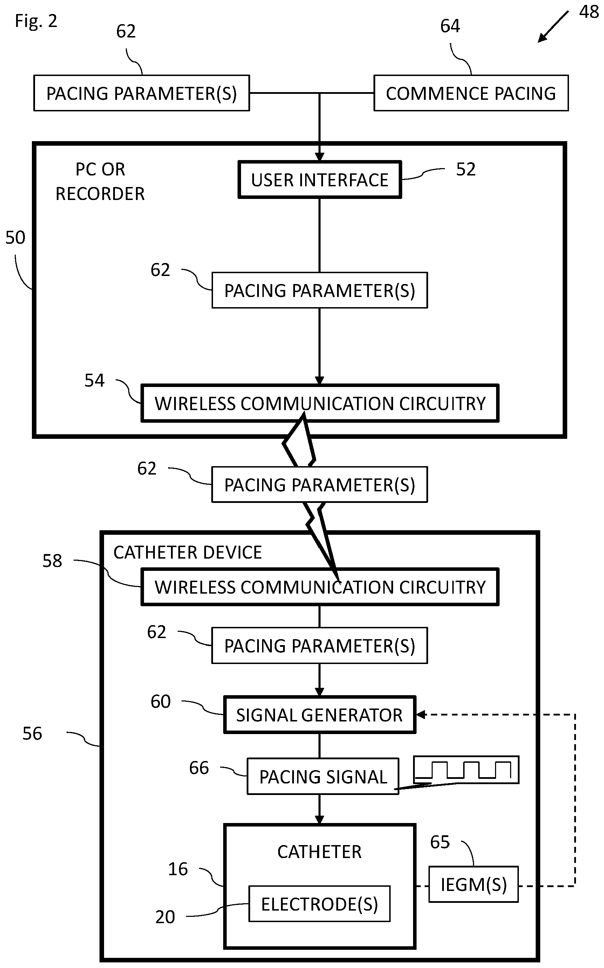

The catheter 16 is configured as a wireless catheter and may share data with the PIU 40 and optionally the recorder 32 and the workstation 42 wirelessly. Wireless pacing using the catheter 16 is described in more detail with reference to Figs. 2-5, which are block diagram views of alternative pacing subsystems in the system of Fig. 1.

Reference is now made to Fig. 2, which shows a pacing subsystem 48 including a device 50 such as the workstation 42 (e.g., a personal computer (PC) or the recorder 32). The device 50 includes a user interface 52 and wireless communication circuitry 54. The user interface 52 may be implemented using software executed by a processor of the device 50. Data may be input via the user interface 52 using any suitable user input device such as a keyboard, mouse, and/or touch sensitive display. The pacing subsystem 48 also includes a catheter device 56, which includes wireless communication circuitry 58, a signal generator 60, and the catheter 16 including electrodes 20. The catheter 16 is configured to be inserted into a cardiac chamber of a living subject. The wireless communication circuitry 58 and signal generator 60 and the catheter 16 are generally connected together using wires, cables, and or other electrical connections.

The user interface 52 is configured to receive one or more pacing parameters 62 set by a user (e.g., the physician 12). The pacing parameter(s) 62 may include any one or more of the following: a pacing signal frequency; a pacing signal duty cycle; a pacing signal amplitude; a pacing signal pulse shape; a pacing signal pulse length; and a given electrode 20 of the catheter 16 to which to apply pacing. The device 50 is configured to store the pacing parameter(s) 62 for later use when the user requests pacing to commence.

The user interface 52 is configured to receive an instruction to commence pacing 64 from the user. The user interface 52 is configured to pass the pacing parameter(s) 62 to the wireless communication circuitry 54 to wirelessly send the pacing parameter(s) 62 to the catheter device 56. The wireless communication circuitry 54 is configured to wirelessly send pacing data based on (e.g., including) the pacing parameter(s) 62 to the catheter device 56 responsively to the instruction to commence pacing.

The wireless communication circuitry 58 is configured to wirelessly receive the pacing parameter(s) 62. The signal generator 60 is configured to receive the pacing parameter(s) 62 from the wireless communication circuitry 58, generate a pacing signal 66 responsively to the received pacing parameter(s) 62, and apply the pacing signal 66 to the selected electrode 20. In some exemplary modes, the pacing signal 66 is a pulsed signal comprising a plurality of pulses.

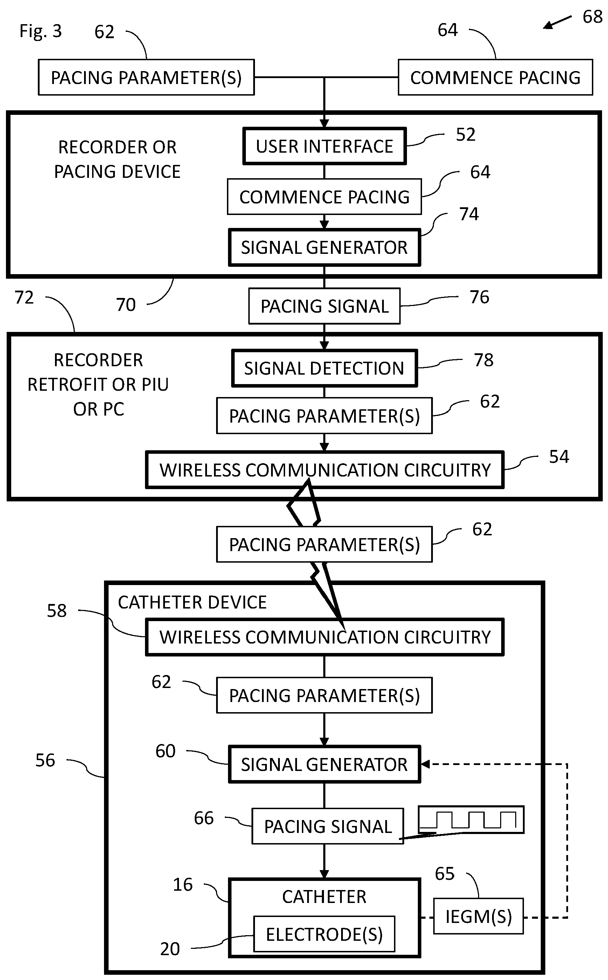

In some exemplary modes, the timing of the pulses of pacing signal 66 may be synchronized with the heart rhythm of the patient. For example, the signal generator 60 may synchronize the pulses of the pacing signal 66 based on intracardiac electrograms (IEGMs) 65 received from electrodes 20 of catheter 16 in the cardiac chamber of the patient. For example, the signal generator 60 may look for the diastolic phase in the IEGMs 65 in which to time the pacing pulses. Reference is now made to Fig. 3, which shows a pacing subsystem 68, which is substantially the same as the pacing subsystem 48 of Fig. 2 except for the differences described below. In particular, the pacing subsystem 68 includes a device 70, a device 72, and the catheter device 56. The catheter device 56 is substantially the same as described with reference to Fig. 2.

The device 70 may be any suitable device, for example, a pacing device or the recorder 32. The device 72 may be any suitable device, for example, the PIU 40, or the workstation 42, or the recorder 32 (which has been retrofitted with hardware and/or software as described in more detail below). In some exemplary modes, the device 70 and the device 72 may be implemented as a single device.

The device 70 includes the user interface 52 and a signal generator 74. The user interface 52 is configured to receive pacing parameter(s) 62 set by a user (e.g., the physician 12). The device 70 is configured to store the pacing parameter(s) 62 for later use when the user requests pacing to commence.

The user interface 52 is configured to receive an instruction to commence pacing 64 from the user. The user interface 52 is configured to pass the pacing parameter(s) 62 to the signal generator 74, which is configured to generate a pacing signal 76 responsively to the instruction to commence pacing. The signal generator 74 may be configured to generate the pacing signal 76 for outputting to a wired port of the device 70 or to the wired port of another device (e.g., the PIU 40), for example, using a legacy pacing subsystem in the device 70.

The device 72 includes signal detection circuitry 78 and wireless communication circuitry 54. The signal detection circuitry 78 is configured to detect the pacing signal 76 and provide the pacing parameter(s) 62 to the wireless communication circuitry 54 to send to the catheter device 56 responsively to detecting the pacing signal 76. The pacing parameter(s) 62 sent by the signal detection circuitry 78 to the wireless communication circuitry 54 may be the same pacing parameter(s) 62 input by the user into the user interface 52. In some exemplary modes, the signal detection circuitry 78 may derive one or more of the pacing parameters 62 from the pacing signal 76.

In some exemplary modes, the timing of the pulses of pacing signal 66 may be synchronized with the heart rhythm of the patient. For example, the signal generator 74 or signal generator 60 may synchronize the pulses of the pacing signal 76 or pacing signal 66 based on intracardiac electrograms (IEGMs) 65 received from electrodes 20 of catheter 16 in the cardiac chamber of the patient. For example, the signal generator 74 or signal generator 60 may look for the diastolic phase in the IEGMs 65 in which to time the pacing pulses. If the IEGMs 65 are sent to the signal generator 74 for analysis, the IEGMs 65 are first digitized by the wireless communication circuitry 58 and sent to the signal generator 74.

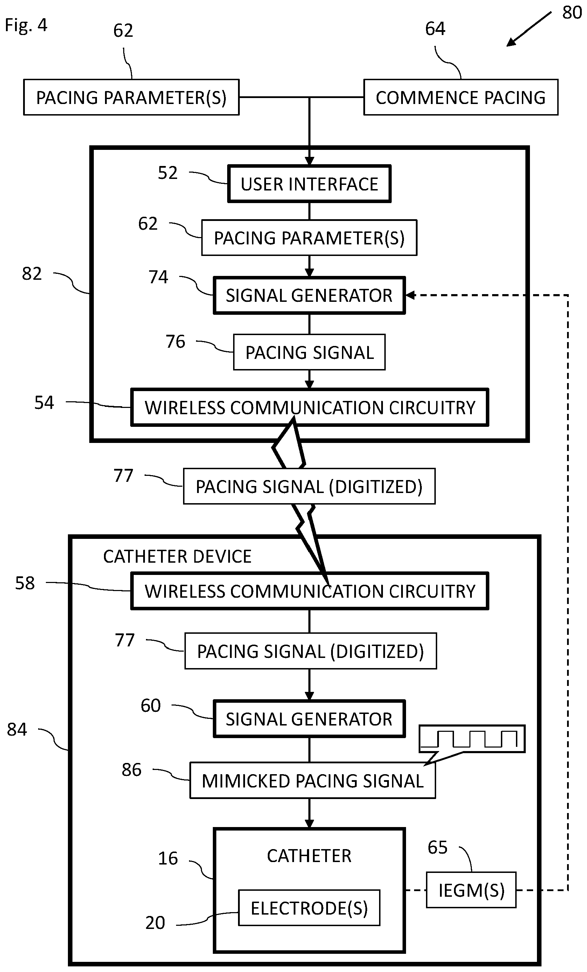

Reference is now made to Fig. 4, which is a view of a pacing subsystem 80. The pacing subsystem 80 includes a device 82 and a catheter device 84. The device 82 may be any suitable device, such as the workstation 42 (e.g., a personal computer (PC) or the recorder 32). The device 82 includes user interface 52, signal generator 74, and wireless communication circuitry 54.

The user interface 52 may be implemented using software executed by a processor of the device 82. Data may be input via the user interface 52 using any suitable user input device such as a keyboard, mouse, and/or touch sensitive display. The user interface 52 is configured to receive pacing parameter(s) 62 set by a user (e.g., the physician 12). The device 50 is configured to store the pacing parameter(s) 62 for later use when the user requests pacing to commence.

The user interface 52 is configured to receive an instruction to commence pacing 64 from the user. The user interface 52 is configured to pass the pacing parameter(s) 62 to the signal generator 74, which is configured to generate the pacing signal 76 responsively to the pacing parameter(s) 62, and provide the pacing signal 76 to the wireless communication circuitry 54 which digitizes pacing signal 76 yielding digitized pacing signal 77. The wireless communication circuitry 54 is configured to wirelessly send pacing data (including the digitized pacing signal 77 and optionally one or more of the pacing parameters 62, e.g., the electrode 20 selected to which to apply the pacing) to the catheter device 84. Therefore, the wireless communication circuitry 54 is configured to wirelessly send the pacing data (e.g., the digitized pacing signal 77) based on the pacing parameter(s) 62 to the catheter device 84 responsively to the instruction to commence pacing 64.

The catheter device 84 includes wireless communication circuitry 58, signal generator 60, and catheter 16 including electrodes 20. The catheter 16 is configured to be inserted into a cardiac chamber of a living subject. The wireless communication circuitry 58 and signal generator 60 and the catheter 16 are generally connected together using wires, cables, and or other electrical connections.

The wireless communication circuitry 58 is configured to wirelessly receive the digitized pacing signal 77 and provide the digitized pacing signal 77 to the signal generator 60. The signal generator 60 is configured to mimic the digitized pacing signal 77 (e.g., perform a digital to analog conversion of the digitized pacing signal 77 and amplify the analog output) yielding a mimicked analog signal 86 and apply the mimicked signal 86 to the electrode 20.

In some exemplary modes, the timing of the pulses of pacing signal 86 may be synchronized with the heart rhythm of the patient. For example, the signal generator 74 or signal generator 60 may synchronize the pulses of the pacing signal 76 or pacing signal 86 based on intracardiac electrograms (IEGMs) 65 received from electrodes 20 of catheter 16 in the cardiac chamber of the patient. For example, the signal generator 74 or signal generator 60 may look for the diastolic phase in the IEGMs 65 in which to time the pacing pulses. If the IEGMs 65 are sent to the signal generator 74 for analysis, the IEGMs 65 are first digitized by the wireless communication circuitry 58 and sent to the signal generator 74.

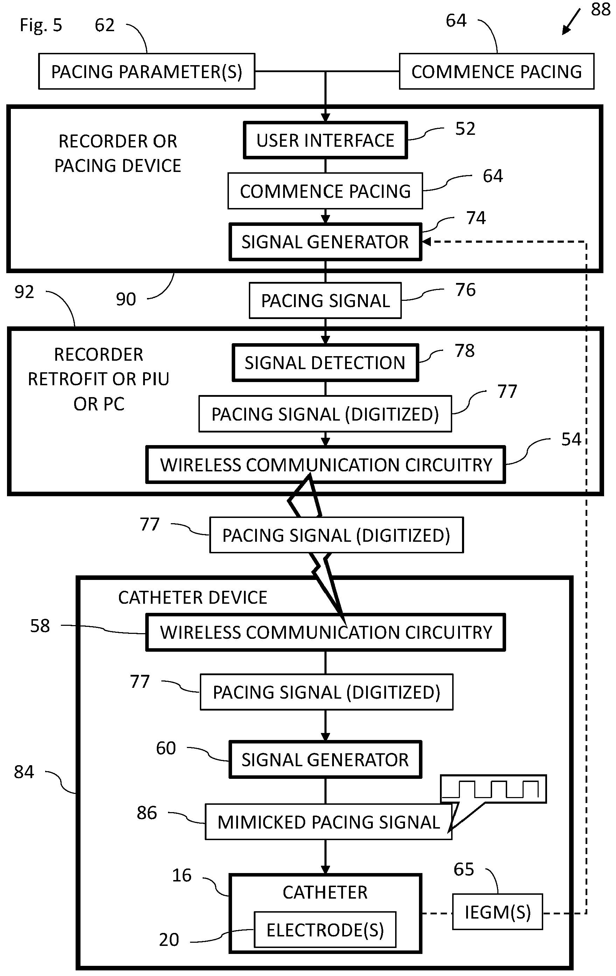

Reference is now made to Fig. 5, which shows a pacing subsystem 88, which is substantially the same as the pacing subsystem 80 of Fig. 4 except for the differences described below. In particular, the pacing subsystem 88 includes a device 90, a device 92, and the catheter device 84. The catheter device 84 is substantially the same as described with reference to Fig. 4.

The device 90 may be any suitable device, for example, a pacing device or the recorder 32. The device 92 may include any suitable device, for example, the PIU 40, or the workstation 42, or the recorder 32 (which has been retrofitted with hardware and/or software as described in more detail below). In some exemplary modes, the device 90 and the device 92 may be implemented as a single device.

The device 90 includes the user interface 52 and signal generator 74. The user interface 52 is configured to receive pacing parameter(s) 62 set by a user (e.g., the physician 12). The device 70 is configured to store the pacing parameter(s) 62 for later use when the user requests pacing to commence.

The user interface 52 is configured to receive an instruction to commence pacing 64 from the user. The user interface 52 is configured to pass the pacing parameter(s) 62 to the signal generator 74, which is configured to generate pacing signal 76 responsively to the instruction to commence pacing 64. The signal generator 74 may be configured to generate the pacing signal 76 for outputting to a wired port of the device 90 or to the wired port of another device (e.g., the PIU 40), for example, using a legacy pacing subsystem in the device 90. In some exemplary modes, when the device 90 is a pacing device, the user interface 52 is configured to receive the pacing parameter(s) 62 set by the user and provide the pacing parameter(s) 62 to the pacing device to generate the pacing signal 76.

The device 92 includes signal detection circuitry 78 and the wireless communication circuitry 54. The signal detection circuitry 78 is configured to detect the pacing signal 76 and digitize the pacing signal 76 yielding digitized pacing signal 77. Therefore, the digitized pacing signal 77 is provided by the signal detection circuitry 78 to the wireless communication circuitry 54 responsively to detecting the pacing signal 76. The wireless communication circuitry 54 is configured to wirelessly send digitized pacing signal 77 to the catheter device 84. As previously mentioned, the catheter device 84 is substantially the same as described with reference to Fig. 4.

In some exemplary modes, the timing of the pulses of pacing signal 86 may be synchronized with the heart rhythm of the patient. For example, the signal generator 74 or signal generator 60 may synchronize the pulses of the pacing signal 76 or pacing signal 86 based on intracardiac electrograms (IEGMs) 65 received from electrodes 20 of catheter 16 in the cardiac chamber of the patient. For example, the signal generator 74 or signal generator 60 may look for the diastolic phase in the IEGMs 65 in which to time the pacing pulses. If the IEGMs 65 are sent to the signal generator 74 for analysis, the IEGMs 65 are first digitized by the wireless communication circuitry 58 and sent to the signal generator 74.

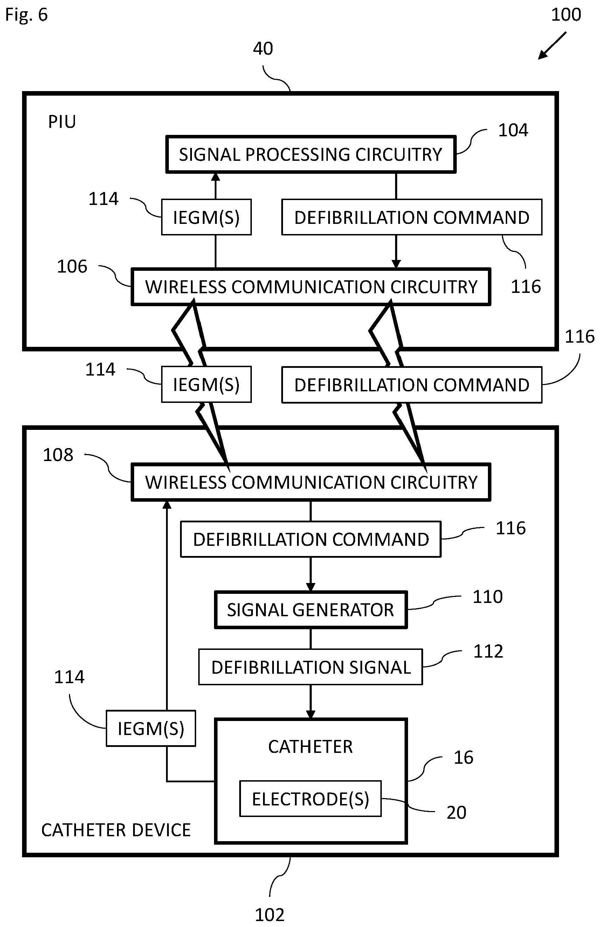

Reference is now made to Fig. 6, which is a view of a defibrillation subsystem 100. The defibrillation subsystem 100 includes the PIU 40 and a catheter device 102. The PIU 40 includes signal processing circuitry 104 and wireless communication circuitry 106. The wireless communication circuitry 106 is configured to wirelessly share data with (e.g., send data to, or receive data from) the catheter device 102.

The catheter device 102 includes the catheter 16. The catheter 16 includes the electrode(s) 20 and is configured to be inserted into the cardiac chamber of a living subject. The catheter device 102 also includes wireless communication circuitry 108 and a signal generator 110. The wireless communication circuitry 108 is configured to wirelessly share the data with the PIU 40. The signal generator 110 is configured to generate a defibrillation signal 112 and apply the defibrillation signal 112 to the electrode(s) 20.

In some exemplary modes, the catheter 16 captures at least one intracardiac electrogram (IEGM) 114 and provides the IEGM(S) 114 to the wireless communication circuitry 108 to digitize and wirelessly send to the wireless communication circuitry 106 of the PIU 40. The wireless communication circuitry 106 of the PIU 40 is configured to wirelessly receive the IEGM(S) 114 captured by the catheter 16. In other exemplary modes, the wireless communication circuitry 106 is configured to receive the IEGM(S) 114 from another catheter.

The signal processing circuitry 104 is configured to analyze the IEGM(S) 114 and cause the signal generator 110 to generate the defibrillation signal 112 responsively to analyzing the IEGM(S) 114.

In some exemplary modes, the signal processing circuitry 104 is configured to send a defibrillation command 116 to the wireless communication circuitry 106, which wirelessly sends the defibrillation command 116 to the wireless communication circuitry 108, which forwards the defibrillation command 116 to the signal generator 110. The signal generator 110 is configured to generate the defibrillation signal 112 responsively to receiving the defibrillation command 116.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

## Claims

1. A medical system (48, 68, 80, 88), comprising a catheter device (56, 84) configured to be inserted into a cardiac chamber of a living subject, and including:
a plurality of electrodes (20), at least one of the electrodes being configured to sense intracardiac electrograms, IEGMs;
first wireless communication circuitry (58) configured to wirelessly receive pacing data based on at least one pacing parameter; and
a first signal generator (60) configured to generate a pacing signal (86) responsively to the received pacing data and apply the pacing signal to one of the electrodes,
wherein the first wireless communication circuitry of the catheter device is configured to wirelessly receive a digitized version (77) of the pacing signal, and
wherein the first signal generator of the catheter device is configured to generate the pacing signal responsively to the digitized version of the pacing signal.

2. The system according to claim 1, wherein the at least one pacing parameter includes any one or more of the following: a pacing signal frequency; a pacing signal duty cycle; a pacing signal amplitude; a pacing signal pulse shape; a pacing signal pulse length; and a given electrode of the catheter device to which to apply pacing.

3. The system according to claim 1 or claim 2, wherein the pacing signal is a pulsed signal comprising a plurality of pulses.

4. The system according to claim 1 or claim 3, wherein:
the first wireless communication circuitry of the catheter device is configured to wirelessly receive the at least one pacing parameter (62); and
the first signal generator of the catheter device is configured to generate the pacing signal (66) responsively to the at least one pacing parameter.

5. The system according to claim 4, further comprising:
a user interface (52) configured to receive an instruction (64) to commence pacing from a user; and
second wireless communication circuitry (54) configured to wirelessly send the at least one pacing parameter to the catheter device responsively to the instruction to commence pacing.

6. The system according to claim 4 or claim 5, further comprising a recorder or a personal computer including:
a user interface (52) configured to receive an instruction (64) to commence pacing from a user;
a second signal generator (74) configured to generate a given pacing signal (76) responsively to the instruction to commence pacing and the at least one pacing parameter;
signal detection circuitry (78) configured to detect the given pacing signal and derive the at least one pacing parameter from the given pacing signal; and
second wireless communication circuitry (54) configured to wirelessly send the at least one pacing parameter to the catheter device.

7. The system according to claim 1, further comprising:
a user interface (52) configured to receive an instruction (64) to commence pacing from a user;
a second signal generator (74) configured to generate a given pacing signal responsively to the instruction to commence pacing and the at least one pacing parameter; and
second wireless communication circuitry (54) configured to digitize the given pacing signal yielding the digitized version of the packing signal and wirelessly send the digitized version of the pacing signal to the catheter device.

8. The system according to claim 7, further comprising:
a pacing device (90) comprising the second signal generator configured to generate the given pacing signal; and
a patient interface unit (92) comprising signal detection circuitry (78) configured to detect the given pacing signal and provide the given pacing signal to the second wireless communication circuitry.

9. The system according to claim 8, wherein the user interface is configured to receive the at least one pacing parameter set by the user and provide the at least one pacing parameter to the pacing device to generate the given pacing signal.

10. The system according to any preceding claim 1, further comprising:
a user interface (52) configured to receive an instruction (64) to commence pacing from a user; and
wireless communication circuitry (54) configured to wirelessly send the pacing data to the catheter device responsively to the instruction to commence pacing.

11. The system according to claim 10, wherein the user interface is configured to receive the at least one pacing parameter (62) set by the user.

## Patentansprüche

1. Medizinisches System (48, 68, 80, 88), umfassend eine Kathetervorrichtung (56, 84), die konfiguriert ist, um in eine Herzkammer eines lebenden Subjekts eingeführt zu werden, und einschließlich:
einer Vielzahl von Elektroden (20), wobei mindestens eine der Elektroden konfiguriert ist, um intrakardiale Elektrogramme, IEGMs, zu erfassen;
einer ersten Drahtloskommunikationsschaltungsanordnung (58), die konfiguriert ist, um Stimulationsdaten basierend auf mindestens einem Stimulationsparameter drahtlos zu empfangen; und
eines ersten Signalgenerators (60), der konfiguriert ist, um ein Stimulationssignal (86) als Reaktion auf die empfangenen Stimulationsdaten zu erzeugen und das Stimulationssignal an eine der Elektroden anzulegen,
wobei die erste Drahtloskommunikationsschaltungsanordnung der Kathetervorrichtung konfiguriert ist, um eine digitalisierte Version (77) des Stimulationssignals drahtlos zu empfangen, und
wobei der erste Signalgenerator der Kathetervorrichtung konfiguriert ist, um das Stimulationssignal als Reaktion auf die digitalisierte Version des Stimulationssignals zu erzeugen.

2. System nach Anspruch 1, wobei der mindestens eine Stimulationsparameter ein beliebiges oder mehrere der Folgenden einschließt: eine Stimulationssignalfrequenz; ein Stimulationssignaltastverhältnis; eine Stimulationssignalamplitude; eine Stimulationssignalimpulsform; eine Stimulationssignalpulslänge; und eine gegebene Elektrode der Kathetervorrichtung, an die eine Stimulation anzulegen ist.

3. System nach Anspruch 1 oder 2, wobei das Stimulationssignal ein gepulstes Signal ist, umfassend eine Vielzahl von Impulsen.

4. System nach Anspruch 1 oder 3, wobei:
die erste Drahtloskommunikationsschaltungsanordnung der Kathetervorrichtung konfiguriert ist, um den mindestens einen Stimulationsparameter (62) drahtlos zu empfangen; und
der erste Signalgenerator der Kathetervorrichtung konfiguriert ist, um das Stimulationssignal (66) als Reaktion auf den mindestens einen Stimulationsparameter zu erzeugen.

5. System nach Anspruch 4, ferner umfassend:
eine Bedienerschnittstelle (52), die konfiguriert ist, um von einem Benutzer eine Anweisung (64) zu empfangen, um mit der Stimulation zu beginnen; und
eine zweite Drahtloskommunikationsschaltungsanordnung (54), die konfiguriert ist, um als Reaktion auf die Anweisung, mit der Stimulation zu beginnen, den mindestens einen Stimulationsparameter drahtlos an die Kathetervorrichtung zu senden.

6. System nach Anspruch 4 oder 5, ferner umfassend ein Aufzeichnungsgerät oder einen Personalcomputer, einschließlich:
einer Bedienerschnittstelle (52), die konfiguriert ist, um von einem Benutzer eine Anweisung (64) zu empfangen, um mit der Stimulation zu beginnen;
eines zweiten Signalgenerators (74), der konfiguriert ist, um als Reaktion auf die Anweisung, mit der Stimulation zu beginnen, und den mindestens einen Stimulationsparameter ein gegebenes Stimulationssignal (76) zu erzeugen;
einer Signalerkennungsschaltungsanordnung (78), die konfiguriert ist, um das gegebene Stimulationssignal zu erkennen und den mindestens einen Stimulationsparameter aus dem gegebenen Stimulationssignal abzuleiten; und
der zweiten Drahtloskommunikationsschaltungsanordnung (54), die konfiguriert ist, um den mindestens einen Stimulationsparameter drahtlos an die Kathetervorrichtung zu senden.

7. System nach Anspruch 1, ferner umfassend:
eine Bedienerschnittstelle (52), die konfiguriert ist, um von einem Benutzer eine Anweisung (64) zu empfangen, um mit der Stimulation zu beginnen;
einen zweiten Signalgenerator (74), der konfiguriert ist, um als Reaktion auf die Anweisung, mit der Stimulation zu beginnen, und den mindestens einen Stimulationsparameter ein gegebenes Stimulationssignal zu erzeugen; und
die zweite Drahtloskommunikationsschaltungsanordnung (54), die konfiguriert ist, um das gegebene Stimulationssignal zu digitalisieren, um die digitalisierte Version des Stimulationssignals zu erhalten, und die digitalisierte Version des Stimulationssignals drahtlos an die Kathetervorrichtung zu senden.

8. System nach Anspruch 7, ferner umfassend:
eine Stimulationsvorrichtung (90), umfassend den zweiten Signalgenerator, der konfiguriert ist, um das gegebene Stimulationssignal zu erzeugen; und
eine Patientenschnittstelleneinheit (92), umfassend eine Signalerfassungsschaltungsanordnung (78), die konfiguriert ist, um das gegebene Stimulationssignal zu erfassen und das gegebene Stimulationssignal der zweiten Drahtloskommunikationsschaltungsanordnung bereitzustellen.

9. System nach Anspruch 8, wobei die Bedienerschnittstelle konfiguriert ist, um den mindestens einen Stimulationsparameter, der durch den Benutzer eingestellt wird, zu empfangen und den mindestens einen Stimulationsparameter an die Stimulationsvorrichtung bereitzustellen, um das gegebene Stimulationssignal zu erzeugen.

10. System nach einem der vorstehenden Ansprüche 1, ferner umfassend:
eine Bedienerschnittstelle (52), die konfiguriert ist, um von einem Benutzer eine Anweisung (64) zu empfangen, um mit der Stimulation zu beginnen; und
eine Drahtloskommunikationsschaltungsanordnung (54), die konfiguriert ist, um die Stimulationsdaten als Reaktion auf die Anweisung, mit der Stimulation zu beginnen, drahtlos an die Kathetervorrichtung zu senden.

11. System nach Anspruch 10, wobei die Bedienerschnittstelle konfiguriert ist, um den mindestens einen Stimulationsparameter (62) zu empfangen, der durch den Benutzer eingestellt wird.

## Revendications

1. Système médical (48, 68, 80, 88), comprenant un dispositif de cathéter (56, 84) conçu pour être inséré dans une chambre cardiaque d'un sujet vivant, et comportant :
une pluralité d'électrodes (20), au moins l'une des électrodes étant configurée pour capter des électrogrammes intracardiaques, IEGM ;
un premier système de circuits de communication sans fil (58) configuré pour recevoir sans fil des données de stimulation en fonction d'au moins un paramètre de stimulation ; et
un premier générateur de signal (60) configuré pour générer un signal de stimulation (86) en réponse aux données de stimulation reçues et appliquer le signal de stimulation à l'une des électrodes,
dans lequel le premier système de circuits de communication sans fil du dispositif de cathéter est configuré pour recevoir sans fil une version numérisée (77) du signal de stimulation, et
dans lequel le premier générateur de signal du dispositif de cathéter est configuré pour générer le signal de stimulation en réponse à la version numérisée du signal de stimulation.

2. Système selon la revendication 1, dans lequel l'au moins un paramètre de stimulation comporte l'un quelconque ou plusieurs quelconques des suivants : une fréquence de signal de stimulation ; un rapport cyclique de signal de stimulation ; une amplitude de signal de stimulation ; une forme d'impulsion de signal de stimulation ; une longueur d'impulsion de signal de stimulation ; et une électrode donnée du dispositif de cathéter à laquelle il faut appliquer une stimulation.

3. Système selon la revendication 1 ou la revendication 2, dans lequel le signal de stimulation est un signal pulsé comprenant une pluralité d'impulsions.

4. Système selon la revendication 1 ou la revendication 3, dans lequel :
le premier système de circuits de communication sans fil du dispositif de cathéter est configuré pour recevoir sans fil l'au moins un paramètre de stimulation (62) ; et
le premier générateur de signal du dispositif de cathéter est configuré pour générer le signal de stimulation (66) en réponse à l'au moins un paramètre de stimulation.

5. Système selon la revendication 4, comprenant en outre :
une interface utilisateur (52) configurée pour recevoir une instruction (64) pour commencer une stimulation en provenance d'un utilisateur ; et
un second système de circuits de communication sans fil (54) configuré pour envoyer sans fil l'au moins un paramètre de stimulation au dispositif de cathéter en réponse à l'instruction pour commencer la stimulation.

6. Système selon la revendication 4 ou la revendication 5, comprenant en outre un enregistreur ou un ordinateur individuel comportant :
une interface utilisateur (52) configurée pour recevoir une instruction (64) pour commencer une stimulation en provenance d'un utilisateur ;
un second générateur de signal (74) configuré pour générer un signal de stimulation donné (76) en réponse à l'instruction pour commencer la stimulation et à l'au moins un paramètre de stimulation ;
un système de circuits de détection de signal (78) configuré pour détecter le signal de stimulation donné et dériver l'au moins un paramètre de stimulation à partir du signal de stimulation donné ; et
un second système de circuits de communication sans fil (54) configuré pour envoyer sans fil l'au moins un paramètre de stimulation au dispositif de cathéter.

7. Système selon la revendication 1, comprenant en outre :
une interface utilisateur (52) configurée pour recevoir une instruction (64) pour commencer une stimulation en provenance d'un utilisateur ;
un second générateur de signal (74) configuré pour générer un signal de stimulation donné en réponse à l'instruction pour commencer la stimulation et à l'au moins un paramètre de stimulation ; et
un second système de circuits de communication sans fil (54) configuré pour numériser le signal de stimulation donné donnant la version numérisée du signal de stimulation et envoyer sans fil la version numérisée du signal de stimulation au dispositif de cathéter.

8. Système selon la revendication 7, comprenant en outre :
un dispositif de stimulation (90) comprenant le second générateur de signal configuré pour générer le signal de stimulation donné ; et
une unité d'interface patient (92) comprenant un système de circuits de détection de signal (78) configuré pour détecter le signal de stimulation donné et fournir le signal de stimulation donné au second système de circuits de communication sans fil.

9. Système selon la revendication 8, dans lequel l'interface utilisateur est configurée pour recevoir l'au moins un paramètre de stimulation défini par l'utilisateur et fournir l'au moins un paramètre de stimulation au dispositif de stimulation pour générer le signal de stimulation donné.

10. Système selon l'une quelconque revendication 1 précédente, comprenant en outre :
une interface utilisateur (52) configurée pour recevoir une instruction (64) pour commencer une stimulation en provenance d'un utilisateur ; et
un système de circuits de communication sans fil (54) configuré pour envoyer sans fil les données de stimulation au dispositif de cathéter en réponse à l'instruction pour commencer la stimulation.

11. Système selon la revendication 10, dans lequel l'interface utilisateur est configurée pour recevoir l'au moins un paramètre de stimulation (62) défini par l'utilisateur.
